# EUROPEAN PATENT APPLICATION

(11) **EP 4 822 381 A1**
(43) Date of publication of application: **14.10.2026**
(21) Application number: 25169940.1
(22) Date of filing: 11.04.2025
(51) Int. Cl.: G01R 33/48, G01R 33/56, G01R 33/561, G01R 33/563, G01R 33/567, A61B 5/00

(54) **CARDIAC MR IMAGING**

(71) Applicant: Siemens Healthineers AG, 91301 Forchheim (DE); King's College London, London WC2R 2LS (GB)
(72) Inventor: CHIRIBIRI, Amedeo, London, SW6 4DJ (GB); GOLDMAN, Oscar, London, W6 9ED (GB); KUNZE, Karl-Philipp, London, SW10 9JR (GB); NEJI, Radhouene, London, N65XP (GB); WHITAKER, John, Hither Green, London SE13 5LT (GB)
(74) Representative: Siemens Healthineers Patent Attorneys

(57) **Abstract**

The invention relates to a method for generating MR images of a patient at an MR imaging system comprising sampling a k-space with a 3-dimensional acquisition schema of k-space with 2 phase encoding directions and one read out direction. The sampling schema includes applying a sequence of inversion pulses to the patient to invert a magnetisation of the patient, wherein sampling the k space is performed with a uniform undersampling of the k space and n different EPI readout shots in the read-out direction after each inversion pulse, with n being => 5. The MR images are reconstructed based on the sampled k space.

## Description

### Technical field

The present invention realtes to a method for generating MR images of a patient using a 3 dimensional acquisition schema of k-space, to an Magnetic Resonsance imaging (MRI) system, a computer program comprising programm code, and a carrier comprising the computer pgrgram. The invention especially realtes to cardiac imaging

### Background

Cardiac imaging with MRI has emerged as a valuable diagnostic tool in the assessment of cardiovascular disease. MRI offers high-resolution images of the heart and its surrounding structures, allowing for detailed evaluation of cardiac anatomy, function, and pathology. MRI imaging of the heart provides high spatial resolution together with excellent soft tissue contrast and functional assessment. Late Gadolinium Enhanced (LGE) MRI is a key component of cardiac MRI exams. It is used to detect and classify myocardial infarction. 3D LGE acquisitions can suffer however from long scan times, which may be difficult for some patients to tolerate. A recent proposal to address this was to produce 3D LGE images in a single breath-hold using Compressed-Sensing (CS) as discussed in Roman Johannes Gertz et al. "Compressed SENSE accelerated 3D single-breathhold late gadolinium enhancement cardiovascular magnetic resonance with isotropic resolution: clinical evaluation". In: Frontiers in Cardiovascular Medicine 10 (Nov. 2023). This was achieved by acquiring highly undersampled images; reducing scan time. However, CS reconstructions can suffer from residual blurring artefacts, particularly at higher undersampling factors.

Accordingly a need exits to avoid the drawbacks mentioned above and to provide an imaging method with reduced artifacts and acceptable image acquisition time.

### Summary

This need is met by the features of the independent claims. Further aspects are described in the dependent claims.

According to a first aspect a method for generating MR images of a patient at an MR imaging system is provided the method comprising the step of sampling a k-space with a three-dimensional acquisition scheme of k-space with two phase encoding directions and one readout direction wherein the sampling includes an application of a sequence of inversion pulses to the patient to invert a magnetization of the patient and the sampling of the k-space is performed with a uniform undersampling of the k-space and n different EPI readout shots in the readout direction after each inversion pulse with n>5. The MR images are then reconstructed based on the sampled k-space.

The combination of the uniform undersampling, the inversion pulses and the EPI readout after each inversion pulse, provides a very effective sampling with reduced acquisition time and acceptable image quality.

Preferably, the MR images are MR images of the heart and the three-dimensional acquisition scheme is used with cardiac gating wherein the n readout shots are acquired within one heartbeat. The imaging method provides especially good results in cardio imaging and the acquisition of the MR images is possible within a single breath-hold.

Preferably the sampling of k-space is carried out after a contrast agent has been applied to the patient and a concentration of the contrast agent has reached an equilibrium in the patient. This is also known as late contrast agent enhanced imaging, also called Late Gadolinium Enhanced, LGE, imaging which is especially useful for certain findings in the heart such as the detection and classification of myocardial infarction. The proposed image acquisition scheme is especially useful in this field of application.

Preferably the k-space is divided in one of the phase encoding directions into n regions and one EPI readout shot from the n shots is acquired from each region within one heartbeat. As a consequence, a uniform undersampling is obtained which is helpful for the image reconstruction.

Additionally, it is possible that in each of the n regions of the k-space S shots are acquired with S>= 3 and the k-space trajectories of shots within one region are shifted relative to one another in at least one of the phase encoding directions using a CAIPIRINHA imaging technique. Here S is preferably in the range between 6 and 10. With this image acquisition and reconstruction technology. It is possible to acquire a 3D whole heart volume during a single breath-hold with satisfying image quality and reduced artifacts. Preferably reconstructing the MR images is performed using a k-space interpolation based technique including GRAPPA (GeneRalized Autocalibrating Partial Parallel Acquisition) using 3D calibration kernels.

Furthermore, the n EPI readout shots can be distributed in k-space such that a center of the k-space is sampled with earlier shots from the n shots and areas outside the center are sampled with later shots from the n shots. The center of k-space which is responsible for the contrast in the image is acquired earlier at a larger magnetization and thus MR signal. As a consequence, the image contrast is improved when the center of k-space is sampled earlier compared to the outer parts of k -space.

Preferably one shot from the n shots is acquired in each of the n regions after each inversion pulse and n is in the range between 5 and 10, more preferably in the range between 6 and 8.

Preferably the inversion time is selected in dependence on an MR signal of the myocardium and more preferably it is selected such that the MR signal of the myocardium is close to zero at image acquisition so that a good contrast is obtained in the images in the area around the myocardium.

Furthermore, an MR imaging system is provided comprising a processing unit and a memory configured to store program code which when carried out by the processing unit causes the processing unit to carry out the above identified steps of acquiring an image.

Furthermore, a computer program comprising program code is provided to be executed by a processing unit of an MR imaging system wherein execution of the program code causes the processing unit to carry out a method as discussed above or as discussed in further detail below.

Finally, a carrier comprising the computer program is provided wherein the carrier is one of an electronic signal, optical signal, radio signal, and computer-readable storage medium.

It is to be understood that the features mentioned above and features yet to be explained below can be used not only in the respective combinations indicated, but also in other combinations or in isolation without departing from the scope of the present invention. Features of the above-mentioned aspects and embodiments described below may be combined with each other in other embodiments unless explicitly mentioned otherwise.

### Brief description of the drawings

Fig. 1 is a schematic view of an MIR imaging system with which a 3 D whole heart volume can be acquired within a single beat-hold.
Fig. 2 shows a schematic view of part of a sampling schema used for acquiring a 3 D volume of the heart
Fig. 3 shows a schematic view of a sampling pattern used to sample k space during the sampling schema of Fig. 2.
Fig. 4 shows an example schematic view of a flowchart comprising steps carried out during the image acquisition od the k-space sampling.

### Detailed description

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are to be illustrative only.

The drawings are to be regarded as being schematic representations, and elements illustrated in the drawings are not necessarily shown to scale. Rather, the various elements are represented such that their function and general purpose becomes apparent to a person skilled in the art. Any connection or coupling between functional blocks, devices, components of physical or functional units shown in the drawings and described hereinafter may be implemented by an indirect connection or coupling. A coupling between components may be established over a wired or wireless connection. Functional blocks may be implemented in hardware, software, firmware, or a combination thereof.

Independent of the grammatical term usage, individuals with male, female or other gender identities are included within the term.

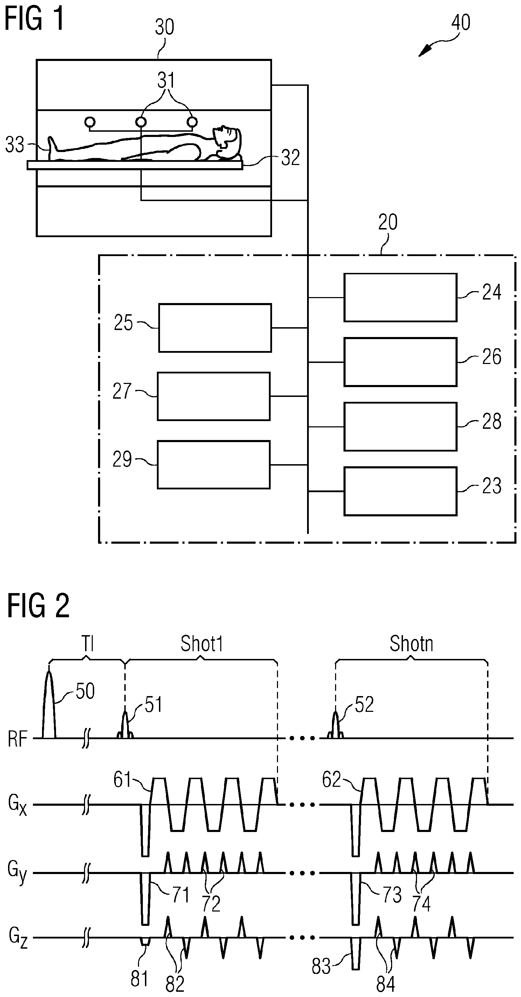

In the following, an MR imaging method will be discussed which provides a 3 D image data set of the heart which is short enough that it can be acquired in a single breath-hold which does not suffer from from artificial image appearance and residual blurring. Figure 1 shows a schematic view of an MRI (Magnetic Resonance Imaging) system 40, which compromises a magnet 30 generating a polarization field B0. An object under examination 33 lying on a table 32 is moved into the center of the MRI system 40 where MR signals after RF excitation can be detected by a receiving coil 31, which can comprise different coil sections wherein each coil section is associated with a corresponding detection channel. By applying RF pulses and magnetic field gradients, the nuclear spins in the object 33, especially in the part located in the receiving coil 31 are exited and the currents induced by the relaxation are detected. The way how MR images are generated and how the MR signals are detected using a sequence of RF pulses and the sequence of magnetic field gradients are known in the art so that a detailed explanation thereof is omitted.

The MRI system comprises a control unit 20 which is used for controlling the MRI system. The control unit 20 comprises a gradient control unit 24 for controlling and switching the magnetic field gradients, an RF control unit 25 for controlling and generating the RF pulses for the imaging sequences. An image sequence control unit 26 is provided which controls the sequence of the applied RF pulses and magnetic field gradients and thus controls the gradient control unit 24 and the RF control unit 25. In a memory 27, computer programs needed for operating the MRI system and the imaging sequences necessary for generating the MR images can be stored together with the generated MR images. The generated MR images can be displayed on a display 28 wherein input unit 29 is provided used by a user of the MR system to control the functioning of the MRI system. A processing unit 23 can coordinate the operation of the different functional units shown in Figure 1 and can comprise one or more processors which can carry out instructions stored on the memory 27. The memory includes the program code to be executed by the processing unit 23.

Fig. 2 shows a structure of a sequence diagram which can be used to generate a 3D data set during a single breath-hold. The imaging sequence uses an inversion recovery-prepared segmented 3D EPI sequence with a skipped-CAIPIRINHA undersampling pattern. An inversion pulse 50 is applied and after the inversion pulse n shots with shorter excitation pulses 51 and 52 are applied to excite the magnetization and after each shot an echo planar imaging, EPI like data acquisition and gradient switching in the readout direction is applied as shown by the gradient switching 61 and 62. The gradient switching in the readout direction (here Gx) generates gradient echos acquired during the switching of Gx. In the phase-encoding directions Gy and Gz a CAIPIRINHA undersampling pattern is used including the gradient switchings 71, 72, 73 and 74 in one of the phase encoding directions together with the gradient switchings 81-84 in the other phase-encoding direction.

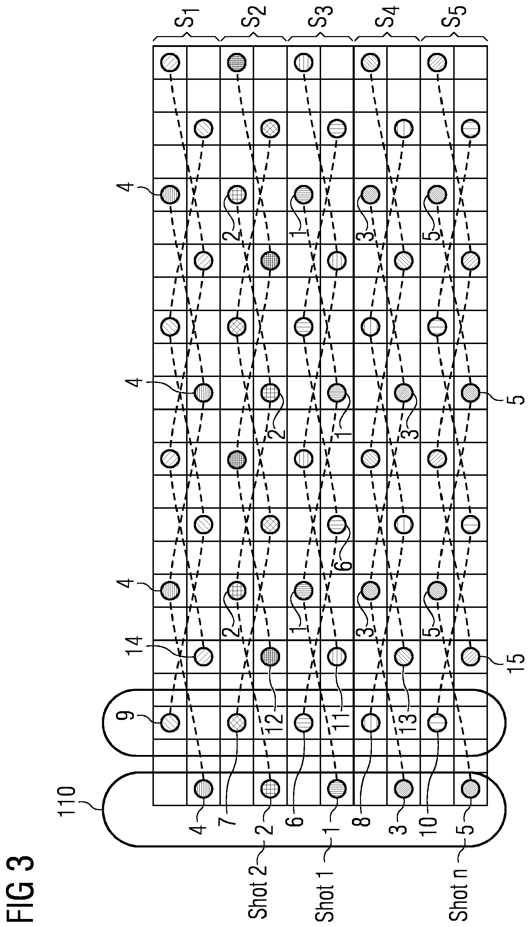

Referring also to Fig. 3, the sampling k-space pattern is shown in more detail. A CAIPIRINHA on the sampling pattern was used and in this scheme a set of several parameters parametrize the sampling mask. The parameter R_{y} describes the undersampling factor in the phase encoding direction, the parameter R_{z} describes the undersampling factor in the partition encoding direction and Δ describes the partition offset between adjacent samples respectively.

In Fig. 3 the numbers shown indicate an order of k-space sampling points wherein all points with number "1" belong to the sampling after the first of the n inversion pulses and all numbers "2" belong to the second data acquisition after the second inversion pulse 52 of Fig. 2. The fields containing a number include a sample wherein the other empty fields were not sampled and the readout direction is running perpendicular into the image plane. Furthermore, the k-space is segmented into several n segments (here S1 to S5) and describes the segmentation factor which determines how the sampling mask is segmented into EPI shots. In the example shown each of the segments S1 to S5 contain three different data acquisitions, however it should be understood that this is only for illustration purposes. Accordingly, in the example indicated in Fig. 3 S is 3 and in each of the segments 3 data acquisition trains are acquired. The image acquisition is then further segmented between different cardiac cycles, here the R-R interval introducing the other parameter n, the number of shots played out after each inversion pulse. This number n is 5 in the example shown in Fig. 3 and the segmentation divides the k-space into n regions, here the five regions and one shot from each region is acquired after each inversion pulse starting from the most central region and moving outward in a centric pattern as shown by the numbers 2, 3, 4 and 5. Accordingly, within each inversion pulse the shots indicated by region 110 are acquired so region 110 includes the number n of shots acquired within one heart beat. Accordingly, the image acquisition is described by five parameters, R_{y}, R_{z}, Δ, S and n which determine the order in which the k-space points are acquired. The image acquisition was tested in patients using a 16 channel coil and a 32 channel spine coil. The imaging sequence was acquired with the following parameters:
a transverse orientation, 2.5mm isotropic resolution providing a matrix of 128 128 56 and a field of view of 320 320 140 mm³. Echo spacing was 0.68 ms, the receiver bandwidth was 1964 Hz/px, a 20° flip angle was used and 1-2-1 binomial water excitation pulse was used for fat suppression. The parameters for (R_{y}, R_{z}, Δ, S, n) were (2, 2, 1, 8, 7) providing an echo train length of 16, an acquisition window of 119 ms and a total undersampling factor of 4. A full 3D heart image could be acquired over 19 heart beats such as one R-R interval.

For the LGE acquisitions shown in Fig. 2 and 3, the inversion time was chosen to null viable myocardium. Furthermore, it is possible to choose a trigger delay such that the acquisition window for an acquisition of the MR signal after the inversion pulse is placed in the quiescent period of the heart, such as the diastole. The image reconstruction could be carried out using an iterative SENSE reconstruction with a stopping criterion being a residual below a predefined threshold fixed to a certain threshold value e.g. 10⁻⁶, or any other value between 10⁻⁸ or 10⁻⁴ The coil sensitivity maps used for the reconstruction can be generated from an external low resolution free breathing gradient echo reference scan, which could preferably be normalised with a body coil.

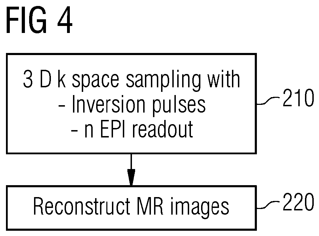

Fig. 4 summarizes some of the steps used for the image acquisition discussed above in connection with Fig. 2 and 3. In step 210 a 3D k-space sampling is carried out using the 2 phase-encoding directions and one readout direction in which a sequence of inversion pulses is applied to invert a magnetization and n different EPI readout shots are used in the readout direction after each inversion pulse. In step 220 the MR images are then reconstructed based on the sampled k-space.

Summarizing the above discussed method enables a fast acquisition of images which allows a 3D single breath-hold image acquisition without the need for very high undersampling factors.

## Claims

1. A method for generating MR images of a patient at an MR imaging system (40) comprising:
- sampling a k-space with a 3-dimensional acquisition schema of k-space with 2 phase encoding directions and one read out direction, including:
- applying a sequence of inversion pulses (50) to the patient to invert a magnetisation of the patient,
- wherein sampling the k space is performed with a uniform undersampling of the k space and n different EPI readout shots in the read out direction after each inversion pulse, with n being => 5,
- reconstructing the MR images based on the sampled k space.

2. The method of claim 1, wherein the MR images are MR images of the heart and the 3-dimensional acquisition schema is used with cardiac gating, wherein the n readout shots are acquired within one heartbeat.

3. The method of claim 2 wherein the k space is divided into in one of the phase encoding directions into n regions (S1 to S5) and one EPI readout shot from the n shots is acquired from each region within one heartbeat.

4. The method of claim 3, wherein in each of the n regions of the k space S shots are acquired, with S being=> 3 and the k space trajectories of shots within one region are shifted relative to one another in one of the phase encoding directions using a CAIPIRINHA imaging technique,

5. The method of claim 4, wherein S is in a range between 6 and 10.

6. The method of claim 4 or 5, wherein reconstructing the MR images is performed using a k-space interpolation based technique including GRAPPA using 3D calibration kernels.

7. The method of any preceding claim, wherein sampling of the k space is carried out after a contrast agent has been applied to the patient and a concentration of the contrast agent has reached an equilibrium in the patient.

8. The method of any preceding claim, the n EPI readout shots are distributed in k space such that a center of the k space is sampled with earlier shots from the n shots and areas outside the center are sampled with later shots from the n shots.

9. The method of claim 3 and 8, wherein one shot from the n shots is acquired in each of the n regions after each inversion pulse.

10. The method of any preceding claim, wherein n is between 5 and 10.

11. The method of any of claims 2 to 10, wherein the inversion time is selected in dependence on an MR signal of the myocardium and preferably an inversion time for the sequence of inversion pulses (50) is selected such that an MR signal of the myocardium is substantially zero during MR signal acquisition.

12. An MR imaging system (40) comprising a processing unit and a memory configured to store program code, which when carried out by the processing unit, cause the processing unit to carry out the steps of:
- sampling a k-space with a 3-dimensional acquisition schema of k-space with 2 phase encoding directions and one read out direction, including:
- applying a sequence of inversion pulses to the patient to invert a magnetisation of the patient,
- wherein sampling the k space is performed with a uniform undersampling of the k space and n different EPI readout shots in the read out direction after each inversion pulse, with n being => 5,
- reconstructing the MR images based on the sampled k space.

13. The MR imaging system of claim 12, wherein the processing unit is configured to carry out a method as mentioned in any of claims 2 to 11.

14. A computer program comprising program code to be executed by a processing unit of an MR imaging system, wherein execution of the program code causes the processing unit to execute a method as mentioned in any of claims 1 to 11.

15. A carrier comprising the computer program of claim 14, wherein the carrier is one of an electronic signal, optical signal, radio signal and computer readable storage medium.
